# EUROPÄISCHE PATENTSCHRIFT

(11) **EP 3 987 413 B1**
(45) Veröffentlichungstag und Bekanntmachung des Hinweises auf die Patenterteilung: **09.09.2026**
(21) Anmeldenummer: 20732763.6
(22) Anmeldetag: 29.05.2020
(51) Int. Cl.: G06F 17/10, G06N 20/00, G06N 3/08, G06N 3/09

(54) **COMPUTERGESTÜTZTES VERFAHREN ZUM ERZEUGEN VON TRAININGSDATEN FÜR EIN NEURONALES NETZ FÜR EINE VORHERSAGE EINER SCHADSTOFFKONZENTRATION**
COMPUTER-ASSISTED METHOD FOR GENERATING TRAINING DATA FOR A NEURAL NETWORK FOR PREDICTING A CONCENTRATION OF POLLUTANTS
PROCÉDÉ ASSISTÉ PAR ORDINATEUR POUR GÉNÉRER DES DONNÉES D'APPRENTISSAGE POUR UN RÉSEAU NEURONAL POUR PRÉDIRE UNE CONCENTRATION DE POLLUANTS

(30) Priorität: 16.08.2019 DE 102019212289
(43) Veröffentlichungstag der Anmeldung: 27.04.2022
(73) Patentinhaber: Siemens Aktiengesellschaft, 80333 München (DE)
(72) Erfinder: JAEGER, Florian Ansgar, 10961 Berlin (DE); MÜLLER, Katrin, 10713 Berlin (DE)
(74) Vertreter: Siemens Patent Attorneys
(86) Internationale Anmeldenummer: PCT/EP2020/064986
(87) Internationale Veröffentlichungsnummer: WO 2021/032332

(56) Entgegenhaltungen:
- KE GU ET AL: "Recurrent Air Quality Predictor Based on Meteorology- and Pollution-Related Factors", IEEE TRANSACTIONS ON INDUSTRIAL INFORMATICS, vol. 14, no. 9, 9 September 2018 (2018-09-09), US, pages 3946 - 3955, XP055741094, ISSN: 1551-3203, DOI: 10.1109/TII.2018.2793950
- DING DAIZONG 17110240010@FUDAN EDU CN ET AL: "Modeling Extreme Events in Time Series Prediction", PROCEEDINGS OF THE 25TH ACM SIGKDD INTERNATIONAL CONFERENCE ON KNOWLEDGE DISCOVERY & DATA MINING , KDD '19, ACM PRESS, NEW YORK, NEW YORK, USA, 25 July 2019 (2019-07-25), pages 1114 - 1122, XP058466144, ISBN: 978-1-4503-6201-6, DOI: 10.1145/3292500.3330896
- BRIAN S. FREEMAN ET AL: "Forecasting air quality time series using deep learning", AIR & WASTE MANAGEMENT ASSOCIATION. JOURNAL, vol. 68, no. 8, 24 May 2018 (2018-05-24), US, pages 866 - 886, XP055740911, ISSN: 1096-2247, DOI: 10.1080/10962247.2018.1459956
- SHUANG LIU ET AL: "Multimodal GAN for Energy Efficiency and Cloud Classification in Internet of Things", IEEE INTERNET OF THINGS JOURNAL, 20 August 2018 (2018-08-20), pages 1 - 8, XP055580521, DOI: 10.1109/JIOT.2018.2866328
- "Designing Sustainable Technologies, Products and Policies : From Science to Innovation", 4 July 2018, SPRINGER INTERNATIONAL PUBLISHING, Cham, ISBN: 978-3-319-66981-6, article FLORIAN ANSGAR JAEGER ET AL: "LCA in Strategic Decision Making for Long Term Urban Transportation System Transformation : From Science to Innovation", pages: 193 - 204, XP055740646, DOI: 10.1007/978-3-319-66981-6_22

## Beschreibung

Die Erfindung betrifft ein Verfahren zum Erzeugen von Trainingsdaten für ein neuronales Netz gemäß dem Oberbegriff des Patentanspruches 1, ein Verfahren zum Training eines neuronalen Netzes gemäß dem Oberbegriff des Patentanspruches 9 sowie ein Verfahren zum Ermitteln einer Schadstoffkonzentration mittels eines neuronalen Netzes gemäß dem Oberbegriff des Patentanspruches 10.

Die Schadstoffbelastung, beispielsweise eine Stickoxidkonzentration, kann innerhalb einiger deutscher Städte für bestimmte Zeiträume oberhalb der zulässigen Grenzwerte liegen.

Zur Sicherstellung einer ausreichenden Luftqualität können Städte mehrere Maßnahmen treffen, beispielsweise Fahrverbote. Allerdings ist es für die Wirksamkeit dieser Maßnahmen erforderlich, dass diese bereits vor einem möglichen Überschreiten der Grenzwerte durchgeführt werden. Hierzu ist eine zuverlässige und möglichst präzise Vorhersage (Prognose) der Schadstoffkonzentration erforderlich.

Grundsätzlich werden Emissionen (Dimension Masse oder Masse pro Länge pro Zeit) und Konzentrationen (Dimension Masse pro Volumen) unterschieden. Die Emission ist die emittierte Masse eines Schadstoffes, beispielsweise eines Verkehrsteilnehmers innerhalb eines Zeitbereiches, beispielsweise einer Stunde. Die Emission kann ebenfalls auf eine Länge (Straßenlänge, Streckenlänge usw.) und einen Zeitbereich bezogen sein, sodass diese hierbei die Dimension Masse pro Länge pro Zeit aufweist. Die Schadstoffkonzentration wird, beispielsweise von einer Messstation, an einem bestimmten Ort innerhalb der Stadt, gemessen. Grundsätzlich sind die Emissionen und Schadstoffkonzentrationen zeitabhängig.

Die Schadstoffkonzentration ist aufgrund der Komplexität der Vorgänge schwer vorherzusagen, sodass hierfür typischerweise neuronale Netze verwendet werden.

Das grundsätzliche Verfahren ist hierbei zweigeteilt. Zunächst wird die Emission mittels eines Modells berechnet. Anschließend wird die Schadstoffkonzentration aus der modellbasiert berechneten Emission mittels des neuronalen Netzes ermittelt.

Hierzu ist ein Training des neuronalen Netzes erforderlich, das heißt es sind Trainingsdaten bezüglich der Schadstoffkonzentration erforderlich. Sinnbildlich muss das neuronale Netz mittels der Trainingsdaten lernen, wie sich die Schadstoffkonzentration aus der Schadstoffemission ergibt. Typischerweise werden zum Trainieren des neuronalen Netzes historische Daten der Schadstoffkonzentration als Trainingsdaten verwendet. Ein derart trainiertes neuronales Netz liefert in Situationen, die häufig auftreten, eine gute Vorhersage. Daher kann die durchschnittliche Schadstoffkonzentration durch diese ausreichend genau vorhergesagt werden.

Problematisch sind Ereignisse oder Situationen hoher Belastung, da diese typischerweise selten sind. Dadurch stehen nur wenig Daten zum Training des neuronalen Netzes zur Verfügung. Durch dieses Problem ist die Vorhersage für die eigentlich interessanten Ereignisse hoher Belastung, das heißt für die seltenen Ereignisse, schlechter.

Aus dem Stand der Technik sind im Wesentlichen zwei Verfahren zur Verbesserung der Vorhersage bezüglich solcher seltenen Ereignisse bekannt.

Erstens können die für das Training verwendeten Daten beziehungsweise Messreihen unterschiedlich gewichtet werden. Beispielsweise wird ein historisches Ereignis hoher Belastung mehrmals verwendet. Nachteilig hieran ist, dass dadurch die Vorhersage der durchschnittlichen Belastung verschlechtert wird. Somit bleibt das eigentliche Problem, dass für Ereignisse hoher Belastung weniger Messreihen beziehungsweise Messdaten und somit Trainingsdaten vorliegen, bestehen.

Zweitens können die Schadstoffemission und Schadstoffkonzentration durch einen vollständigen modellbasierten Ansatz berechnet werden. Dies ist ein großer Aufwand und zudem sind nicht alle Abhängigkeiten bekannt. Die bekannten Verfahren liefern daher typischerweise zu geringe Werte für die Schadstoffkonzentration. BRIAN S. FREEMAN ET AL: "Forecasting air quality time series using deep learning", AIR & WASTE MANAGEMENT ASSOCIATION. JOURNAL, Bd. 68, Nr. 8, 24. Mai 2018 (2018-05-24), Seiten 866-886, XP055740911, US ISSN: 1096-2247, DOI: 10.1080/10962247.2018.1459956 offenbart Deep-Learning-Techniken für die Vorhersage von Luftverschmutzungszeitreihen.

Der vorliegenden Erfindung liegt die Aufgabe zugrunde, ein verbessertes Training eines neuronalen Netzes, welches zur Ermittlung einer Schadstoffkonzentration aus einer Schadstoffemission vorgesehen ist, bereitzustellen.

Die Aufgabe wird durch ein Verfahren zum Erzeugen von Trainingsdaten für ein neuronales Netz mit den Merkmalen des unabhängigen Patentanspruches 1, durch ein Verfahren zum Training eines neuronalen Netzes mit den Merkmalen des unabhängigen Patentanspruches 9, sowie durch ein Verfahren zum Ermitteln einer Schadstoffkonzentration mit den Merkmalen des unabhängigen Patentanspruches 10 gelöst. In den abhängigen Patentansprüchen sind vorteilhafte Ausgestaltungen und Weiterbildungen der Erfindung angegeben.

Das erfindungsgemäße computergestützte Verfahren zum Erzeugen von Trainingsdaten für ein neuronales Netz, wobei das neuronale Netz zum Ermitteln einer Schadstoffkonzentration aus wenigstens einer Schadstoffemission ausgebildet ist, umfasst wenigstens die folgenden Schritte:
- Bereitstellen wenigstens einer Messreihe der Schadstoffkonzentration mit wenigstens einem Messwert, der oberhalb eines festgelegten Schwellenwertes ist;
- Bereitstellen wenigstens einer Messreihe für eine zur gemessenen Schadstoffkonzentration zugehörigen physikalischen Messgröße, insbesondere einer Temperatur, einer Windgeschwindigkeit und/oder einer Verkehrsstärke;
- Bereitstellen eines Modells, wobei das Modell einen Zusammenhang zwischen der Messgröße und der Schadstoffemission modelliert;
- Berechnen eines ersten Wertes *E*₀ der Schadstoffemission mittels des Modells, wobei hierzu wenigstens ein zu einem (ursprünglichen) Wert *C*₀ der bereitgestellten gemessenen Schadstoffkonzentration zugehöriger Messwert der Messgröße verwendet wird;
- Berechnen eines zweiten Wertes *E*₁ der Schadstoffemission mittels des Modells, wobei hierzu der für das Berechnen des ersten Wertes *E*₀ der Schadstoffemissionen verwendete Messwert der Messgröße numerisch verändert wird; und
- Erzeugen einer synthetischen Messreihe als Trainingsdaten durch eine Veränderung Δ*C* des Wertes *C*₀ der bereitgestellten gemessenen Messreihe der Schadstoffkonzentrationen, wobei die Veränderung Δ*C* mittels der relativen Änderung Δ*E*/*E*₀ der berechneten Werte der Schadstoffemissionen erfolgt.

Das erfindungsgemäße Verfahren zum Erzeugen von Trainingsdaten stellt Daten beziehungsweise eine Zeitreihe der Schadstoffkonzentration bereit, mittels welchem das neuronale Netz trainiert werden kann. Das Training kann mittels bekannter Verfahren, beispielsweise Deep-Learning, erfolgen.

Das neuronale Netz (englisch: Artificial Neural Network) ist hierbei dazu vorgesehen oder dazu ausgebildet, aus einer Schadstoffemission eine Schadstoffkonzentration zu ermitteln. Die Schadstoffemission beziehungsweise die Schadstoffemissionen werden mittels des Modells berechnet.

Im ersten Schritt des erfindungsgemäßen Verfahrens zum Erzeugen der Trainingsdaten wird eine Messreihe einer Schadstoffkonzentration bereitgestellt, wobei wenigstens ein Wert beziehungsweise Messwert der Schadstoffkonzentration oberhalb des festgelegten Schwellenwertes ist. Mit anderen Worten wird eine Messreihe bereitgestellt, die zu einer wenigstens zu einem Zeitpunkt hohen Schadstoffkonzentration und somit zu einer hohen Schadstoffbelastung korrespondiert. Somit lag ein seltenes Ereignis einer hohen Schadstoffbelastung vor.

Der Schwellenwert ist typischerweise durch einen Grenzwert festgelegt, beispielsweise 200 Mikrogramm pro Kubikmeter (µg/m³) für Stickoxid. Die Messreihe ist eine zeitliche **Ab**folge (kontinuierlich oder diskret) von Messwerten der Schadstoffkonzentration, beispielsweise in der Einheit µg/m³. Die Messreihe weist einen oder mehrere Messwerte auf, wobei jeder Messwert zu einem bestimmten Zeitpunkt erfasst worden ist. Der Zeitpunkt kann ebenfalls ein Zeitbereich sein, sodass für den Zeitbereich ein Messwert erfasst oder ermittelt wurde. Beispielsweise wird für jede Stunde ein Messwert der Schadstoffkonzentration ermittelt, beispielsweise durch eine oder mehrere Messungen. Mit anderen Worten wird beispielsweise für jede Stunde eines Tages ein Messwert der Schadstoffkonzentration erfasst. Die zeitliche geordnete Abfolge dieser erfassten Messwerte bildet dann eine exemplarische Messreihe der Schadstoffkonzentration aus.

Im zweiten Schritt des erfindungsgemäßen Verfahrens zum Erzeugen der Trainingsdaten wird wenigstens eine Messreihe einer physikalischen Messgröße bereitgestellt. Hierbei ist die Messgröße eine physikalische Größe, beispielsweise eine Temperatur, eine Windgeschwindigkeit und/oder eine Verkehrsstärke beziehungsweise Verkehrsdichte. Die Messgröße ist zur bereitgestellten gemessenen Schadstoffkonzentration zugehörig, das heißt, dass für jeden Zeitpunkt ein Messwert der Schadstoffkonzentration und ein Messwert der Messgröße vorliegt. Mehrere Messgrößen und entsprechende Messreihen können vorgesehen sein.

Beispielsweise wird für jede Stunde eines Tages eine durchschnittliche Schadstoffkonzentration und die bei der jeweiligen durchschnittlichen Schadstoffkonzentration vorliegende und somit zugehörige durchschnittliche Temperatur, Windgeschwindigkeit und/oder Verkehrsstärke erfasst. Mit anderen Worten werden wenigstens zwei Messgrößen zeitlich erfasst, die Schadstoffkonzentration und die physikalische Messgröße, beispielsweise die Temperatur, die Windgeschwindigkeit und/oder die Verkehrsstärke, die bei der gemessenen Schadstoffkonzentration vorliegen beziehungsweise vorlagen. Die Messgröße ist deshalb von Bedeutung, da diese oder mehrere Messgrößen, wie beispielsweise die Temperatur, die Windgeschwindigkeit und/oder die Verkehrsstärke, die Schadstoffkonzentration grundsätzlich beeinflussen, das heißt die Schadstoffkonzentration ist von der einen oder mehreren Messgrößen abhängig. So kann die Schadstoffkonzentration an einer Messstation innerhalb einer Stadt entscheidend von der Windrichtung und/oder der Verkehrsstärke abhängen.

Im dritten Schritt des erfindungsgemäßen Verfahrens zum Erzeugen der Trainingsdaten wird ein Modell bereitgestellt, wobei das Modell einen Zusammenhang (Abhängigkeit) zwischen der Messgröße und der Schadstoffemission modelliert beziehungsweise beschreibt. Mittels des Modells kann somit die Schadstoffemission, beispielsweise eines Verkehrsteilnehmers, in Abhängigkeit der Messgröße, beispielsweise der Temperatur, der Windgeschwindigkeit und/oder der Verkehrsstärke, berechnet werden. Typischerweise sind diese Modelle komplex und domainbasiert. Das Modell weist somit wenigstens eine Eingangsgröße und wenigstens eine Ausgangsgröße auf, wobei die Eingangsgröße die Messgröße ist, und die Ausgangsgrößen die Schadstoffemission ist.

Im vierten Schritt des erfindungsgemäßen Verfahrens zum Erzeugen der Trainingsdaten wird ein erster Wert *E*₀ der Schadstoffemission mittels des Modells berechnet. Hierzu wird wenigstens eine zu einem Wert *C*₀ der bereitgestellten gemessenen Schadstoffkonzentration zugehöriger Messwert der Messgröße verwendet. Mit anderen Worten wird der zum Wert *C*₀ der bereitgestellten gemessenen Schadstoffkonzentration zugehörige Wert der Messgröße, beispielsweise der zum Wert der Schadstoffkonzentration zugehörige Wert der Temperatur, als Eingangsgröße des Modells verwendet. Hieraus berechnet das Modell dann den ersten Wert *E*₀ der Schadstoffemission. Beispielsweise werden Temperatur, Windgeschwindigkeit und/oder Verkehrsstärke in das Modell als Eingangsgrößen gegeben, woraus das Modell dann die erste Schadstoffemission *E*₀ berechnet.

Im fünften Schritt des erfindungsgemäßen Verfahrens zum Erzeugen der Trainingsdaten wird ein zweiter Wert *E*₁ der Schadstoffemission mittels des Modells berechnet. Hierzu wird der für das Berechnen des ersten Wertes *E*₀ der Schadstoffemissionen verwendete Messwert der Messgröße numerisch verändert. Mit anderen Worten wird die zweite Schadstoffemission *E*₁ für einen veränderten Wert der Messgröße, beispielsweise für einen veränderten Wert der Temperatur, der Windgeschwindigkeit und/oder der Verkehrsstärke berechnet. Der veränderte Wert der Messgröße oder dementsprechend die veränderte Messreihe der Messgröße wird somit als Eingangsgröße in das Modell gegeben. Dadurch wird der zweite Wert der Schadstoffemission *E*₁ beziehungsweise eine zweite Schadstoffemission beziehungsweise eine zweite Zeitreihe der Schadstoffemission berechnet. In diesem Sinne korrespondiert der zweite Wert der Schadstoffemission *E*₁ zu einer synthetischen Schadstoffemission, die beim entsprechenden veränderten Wert der Messgröße, beispielsweise bei einer veränderten Temperatur, einer veränderten Windgeschwindigkeit und/oder einer veränderten Verkehrsstärke vorliegen würde. Hierbei ist es vorteilhaft den Wert der Messgröße nur geringfügig zu verändern. Beispielsweise ist die relative Änderung des Wertes der Messgröße bevorzugt kleiner als 10 Prozent.

Im sechsten Schritt des erfindungsgemäßen Verfahrens wird eine neue, weitere oder synthetische Messreihe erzeugt, die dem Trainingsdatensatz zugrunde liegt. Mit anderen Worten umfasst der Trainingsdatensatz die neue Messreihe, wobei das neuronale Netzwerk mittels der neuen Messreihe trainierbar ist. Die neue Messreihe wird durch eine Veränderung Δ*C* des Wertes *C*₀ der bereitgestellten gemessenen Messreihe der Schadstoffkonzentrationen erzeugt, wobei die Veränderung Δ*C* mittels der relativen Änderung Δ*E*/*E*₀ = (*E*₁ - *E*₀)/*E*₀ der berechneten Werte der Schadstoffemissionen erfolgt. Da der neuen Messreihe der zweite (synthetisch) berechnete Wert *E*₁ der Schadstoffemission zugrunde liegt, und der zweite Wert *E*₁ eben nicht auf einen gemessenen Wert der Messgröße basiert, kann die neue beziehungsweise weitere Messreihe der Schadstoffkonzentration ebenfalls als synthetische Messreihe bezeichnet werden. Mit anderen Worten ist die neu erzeugte Messreihe im Hinblick auf die bereitgestellte gemessene Messreihe nicht gemessen worden, sondern mittels des beschriebenen Verfahrens synthetisch erzeugt.

Durch die vorliegende Erfindung kann somit eine Mehrzahl von synthetischen Messreihen der Schadstoffkonzentration erzeugt werden, mit welchem das neuronale Netz, wie bereits mit der ursprünglich gemessenen Messreihe der Schadstoffkonzentration, trainiert werden kann. Da die ursprüngliche bereitgestellte gemessene Messreihe der Schadstoffkonzentration zu einem selten Ereignis hoher Belastung korrespondiert - was durch den Schwellenwert des ersten Schrittes des vorliegenden Verfahrens sichergestellt ist, können somit mehrere Messreihen von seltenen Ereignissen hoher Belastung synthetisch erzeugt werden. Wird das neuronale Netz mittels dieser neu erzeugten synthetischen Messreihen trainiert, so wird die Vorhersage des neuronalen Netzes bezüglich der genannten seltenen Ereignisse verbessert, ohne das eine Verschlechterung des durchschnittlichen Verhaltens zu erwarten ist.

Mit anderen Worten ermöglicht die vorliegende Erfindung es dem neuronalen Netz von einem umfangreicheren Trainingsdatensatz zu lernen. Dadurch wird die Vorhersage des neuronalen Netzes bezüglich der seltenen, aber relevantesten Ereignisse hoher Belastung verbessert.

Weiterhin ist die Integration des Vorhersagealgorithmus in bereits bestehende Modelle nicht komplexer als die Verwendung herkömmlicher Algorithmen für neuronale Netze. Das ist deshalb der Fall, da diese zwar verbessert werden, aber in ihrer Struktur unverändert bleiben. Mit anderen Worten betrifft die vorliegende Erfindung zunächst das Trainieren des neuronalen Netzes beziehungsweise das Erzeugen eines zugehörigen Trainingsdatensatzes beziehungsweise ein Erweitern eines bereits bestehenden Trainingsdatensatzes.

Im Vergleich zu einer Gewichtung von Messwerten kann ebenfalls eine wesentlich bessere Datenbasis erzeugt werden. Verglichen mit einem vollständigen modellbasierten Ansatz sind Aufwand und Datenanforderung deutlich geringer. Weiterhin muss das Modell nicht online für eine Vorhersage betrieben werden, sondern muss lediglich für die spezifischen und relevanten Ereignisse oder Szenarien zum Trainieren des neuronalen Netzes laufen. Dadurch kann vorteilhafterweise Rechenzeit eingespart werden. Ein Onlinebetrieb kann jedoch vorgesehen sein.

Die vorliegende Erfindung ermöglicht somit eine genauere Vorhersage bei einem geringeren Aufwand und reduzierten Datenanforderungen.

Das erfindungsgemäße computergestützte Verfahren zum Trainieren eines neuronalen Netzes, wobei das neuronale Netz zum Ermitteln einer Schadstoffkonzentration aus wenigstens einer Schadstoffemission ausgebildet ist, ist gekennzeichnet dadurch, dass zum Trainieren des neuronalen Netzes ein gemäß der vorliegenden Erfindung und/oder einer ihrer Ausgestaltungen erzeugter Trainingsdatensatz verwendet wird.

Es ergeben sich zum erfindungsgemäßen Verfahren zum Erzeugen der Trainingsdaten gleichartige und gleichwertige Vorteile und Ausgestaltungen.

Das erfindungsgemäße computergestützte Verfahren zum Ermitteln einer Schadstoffkonzentration mittels eines neuronalen Netzes und mittels eines Modells, wobei das neuronale Netz zum Ermitteln einer Schadstoffkonzentration aus wenigstens einer Schadstoffemission ausgebildet und gemäß der vorliegenden Erfindung und/oder einer ihrer Ausgestaltungen trainiert ist, wobei das Modell einen Zusammenhang zwischen einer physikalischen Messgröße, insbesondere einer Temperatur, einer Windgeschwindigkeit und/oder einer Verkehrsstärke, und der Schadstoffemission modelliert, ist gekennzeichnet durch folgende Schritte:
- Berechnen eines Wertes der Schadstoffemission mittels des Modells, wobei hierzu wenigstens ein Messwert der Messgröße verwendet wird; und
- Ermitteln der Schadstoffkonzentration aus dem berechneten Wert der Schadstoffemission mittels des neuronalen Netzes.

Dadurch wird vorteilhafterweise eine Vorhersage für die Schadstoffkonzentration bereitgestellt. Die Vorhersage entspricht der ermittelten Schadstoffkonzentration. Basierend auf der ermittelten Schadstoffkonzentration können technische Maßnahmen vorgesehen sein, die zu einer tatsächlichen Reduzierung der Schadstoffkonzentration führen. Die Vorhersage kann alternativ oder ergänzend bereits solche automatisierten Maßnahmen vorsehen und/oder diese vorschlagen. Beispielsweise könnte als eine solche Maßnahme der Verkehr durch eine entsprechende Ampelschaltung umgeleitet werden und/oder Straßen vollständig gesperrt werden. Zudem könnten automatisiert und basierend auf der erfindungsgemäßen Vorhersage mehr Busse und/oder Straßenbahnen zur Verfügung gestellt werden.

Es ergeben sich zum erfindungsgemäßen Verfahren zum Erzeugen der Trainingsdaten beziehungsweise zum erfindungsgemäß trainierten neuronalen Netz gleichartige und gleichwertige Vorteile und Ausgestaltungen.

Gemäß einer vorteilhaften Ausgestaltung der Erfindung erfolgt die Veränderung Δ*C* des wenigstens einen Wertes *C*₀ der bereitgestellten Messreihe der Schadstoffkonzentrationen zusätzlich mittels eines verkehrsbedingten Anteils *α* an der Schadstoffkonzentration.

Mit anderen Worten wird der verkehrsbedingte Anteil an der Schadstoffkonzentration berücksichtigt. Typischerweise setzt sich die Schadstoffkonzentration eines Schadstoffes, beispielsweise von Stickoxid, aus mehreren Anteilen zusammen. Die Anteile sind hauptsächlich der Verkehr, die Gebäude und die Industrie sowie die Energieerzeugung. Der Anteil des Verkehrs, das heißt der verkehrsbedingte Anteil *α*, ist typischerweise bekannt. Beispielsweise durch einen Vergleich mit einer weiteren nicht so stark durch den Verkehr belasteten Messstation. Dadurch kann vorteilhafterweise in effizienter Weise von den Schadstoffemissionen auf die Schadstoffkonzentrationen geschlossen werden, ohne dass eine explizite und aufwendige Berechnung oder Ermittlung erforderlich ist. Dieser approximative heuristische Ansatz ermöglicht somit eine effiziente Ermittlung der Schadstoffkonzentrationen aus den Schadstoffemissionen und somit zur Bereitstellung beziehungsweise Erzeugung des Trainingsdatensatzes.

In einer vorteilhaften Ausgestaltung der Erfindung erfolgt die Veränderung Δ*C* des wenigstens einen Wertes *C*₀ der bereitgestellten Messreihe der Schadstoffkonzentration mittels Δ*C*/*C*₀ *= α*Δ*E*/*E*₀.

Mit anderen Worten wird bevorzugt eine lineare Abhängigkeit zwischen der relativen Änderung der Schadstoffemissionen und der relativen Änderung der Schadstoffkonzentrationen verwendet. Die relative Änderung der Schadstoffemissionen wird gemäß der vorliegenden Erfindung durch das Modell ermittelt. Das heißt, dass ausgehend von einem Messwert der Messgröße, beispielsweise der Temperatur, der Windgeschwindigkeit und/oder der Verkehrsstärke, diese Messgröße in ihrem Wert verändert wird, und eine zum veränderten Messwert zugehörige neue Schadstoffemission ermittelt und die relative Änderung zwischen der neuen Schadstoffemission (zweite Schadstoffemission) und der zum ursprünglichen Messwert der Messgröße zugehörigen Schadstoffemission (erste Schadstoffemission) berechnet wird. Die Schadstoffkonzentration, welche für das Trainieren des neuronalen Netzes erforderlich ist, wird mittels des verkehrsbedingten Anteils *α* aus der derart ermittelten relativen Änderung der Schadstoffemission bestimmt. Dies wird insbesondere für jeden Wert beziehungsweise Zeitpunkt der ursprünglichen Messreihe der Schadstoffkonzentrationen durchgeführt. Mit anderen Worten wird jeder Wert *C*₀ der Messreihe der Schadstoffkonzentration um ein typischerweise verschiedenes Δ*C* verändert. Der Wert *C*₁ der derart neu gebildeten synthetischen Messreihe der Schadstoffkonzentration wird demnach für jeden Zeitpunkt *t* durch *C*₁(*t*) *= C*₀(*t*) *+ ΔC(t)* beziehungsweise für diskrete Zeitwerte *tₙ* durch *C*₁(*tₙ*) *= C*₀(*tₙ* ) *+* Δ*C*(*tₙ*) ermittelt. Es können ebenfalls nur Teilbereiche der Messreihe der Schadstoffkonzentration derart verändert werden, insbesondere lediglich ein Wert beziehungsweise Zeitpunkt der genannten Messreihe. Weitere mathematisch äquivalente Formulierungen und/oder Änderungen können vorgesehen sein.

Gemäß einer vorteilhaften Ausgestaltung der Erfindung wird ein verkehrsbedingter Anteil *α* im Bereich von 0,3 bis 0,5 verwendet.

Mit anderen Worten hat der Verkehr, welcher beispielsweise den Straßenverkehr umfasst, an der Schadstoffkonzentration, beispielsweise an einer Messstation an einer Straße, einen Anteil im Bereich von 0,3 bis 0,5. Besonders bevorzugt ist ein hoher lokaler verkehrsbedingter Anteil (Verkehrsanteil). Der verkehrsbedingte Anteil *α* ist grundsätzlich von den Umständen des Einzelfalls, beispielsweise der Stadt, der Straße, des Standortes der Messstation usw., abhängig. Dennoch hat sich gezeigt, dass hohe lokale verkehrsbedingte Anteile, bestenfalls in Kombination mit einem möglichst homogen urbanen Hintergrund, zur Ermittlung der relativen Änderung der Schadstoffkonzentration aus der relativen Änderung der Schadstoffemission besonders gut geeignet ist.

In einer vorteilhaften Weiterbildung der Erfindung wird eine Stickoxidkonzentration als Schadstoffkonzentration und eine Stickoxidemission als Schadstoffemission verwendet wird.

Mit anderen Worten ist der betrachtete Schadstoff Stickstoffmonoxid und/oder Stickstoffdioxid (zusammenfassend NO_{X}). Weitere Stickoxidverbindungen können alternativ oder ergänzend vorgesehen sein. Ebenfalls können weitere Schadstoffe alternativ oder ergänzend vorgesehen sein. So kann die vorliegende Erfindung für eine Mehrzahl von Schadstoffen oder Schadstoffklassen verwendet werden. Insbesondere ebenfalls für Partikelklassen von Schadstoffen, beispielsweise PM₁₀ und/oder PM_{2.5}.

Gemäß einer vorteilhaften Ausgestaltung der Erfindung wird als physikalische Messgröße eine Temperatur, eine Windgeschwindigkeit und/oder eine Verkehrsstärke verwendet.

Die Temperatur, die Windgeschwindigkeit und/oder die Verkehrsstärke sind relevante Größen, insbesondere die Temperatur und die Verkehrsstärke, die die zeitliche und räumliche Verteilung und Ausbreitung der Schadstoffemission und somit die Bildung der Schadstoffkonzentration, beispielsweise am Ort der Messstation, maßgeblich beeinflussen und/oder bestimmen. Mit anderen Worten hängt die Schadstoffkonzentration, die beispielsweise zu einem Zeitpunkt beziehungsweise innerhalb eines Zeitbereiches, durch eine Messstation gemessen wird, von der Temperatur, der Windgeschwindigkeit und/oder der Verkehrsstärke ab. Grundsätzlich ist die Windgeschwindigkeit ein Vektorfeld, welches typischerweise eine bezüglich der Erdoberfläche horizontale und vertikale Komponente aufweist. Vorliegend können ebenfalls Teilgrößen der Windgeschwindigkeit, beispielsweise eine Windrichtung (horizontale Komponente), der Betrag der Windgeschwindigkeit und/oder eine Windstärke (Kategorisierung in Geschwindigkeitsintervalle), als Messgröße verwendet werden. Weitere physikalische Messgrößen können alternativ oder ergänzend vorgesehen sein.

In einer vorteilhaften Ausgestaltung der Erfindung wurden die Messreihe der Schadstoffkonzentration und die Messreihe der Messgröße mittels einer Messstation innerhalb einer Stadt erfasst.

Das ist deshalb bevorzugt, da hohe Schadstoffkonzentrationen innerhalb von Städten auftreten und dort eine Vielzahl von Menschen direkt betroffen sind. Dort sind somit Maßnahmen zur Vermeidung solcher hohen Schadstoffkonzentrationen besonders erforderlich. Die vorliegende Erfindung und/oder eine ihrer Ausgestaltungen kann hierzu, durch eine verbesserte Vorhersage, die durch ein verbessert trainiertes neuronales Netz ermöglicht wird, einen entscheidenden Beitrag leisten.

Bevorzugt werden für das Verfahren gemäß der vorliegenden Erfindung und/oder einer ihrer Ausgestaltungen die genannten Messreihen erfasst.

Gemäß einer bevorzugten Ausgestaltung der Erfindung wird als Modell ein domainbasiertes Modell verwendet.

Insbesondere umfasst das Modell die verkehrsspezifischen Schadstoffemissionen. Mit anderen Worten sind mittels des Modells die Schadstoffemissionen des Verkehrs, beispielsweise in einem Bereich einer Stadt und/oder an einer Straße, berechenbar. Das Modell modelliert somit die verkehrsspezifischen Schadstoffemissionen.

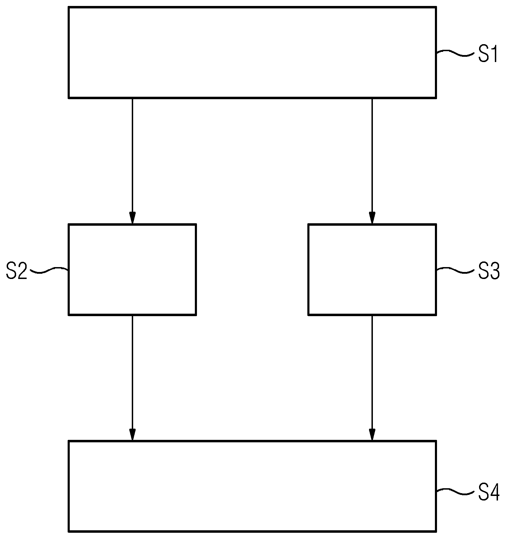

Weitere Vorteile, Merkmale und Einzelheiten der Erfindung ergeben sich aus den im Folgenden beschriebenen Ausführungsbeispielen sowie anhand der Zeichnung. Dabei zeigt die einzige Figur ein schematisiertes Ablaufdiagramm einer Ausgestaltung der Erfindung.

Gleichartige, gleichwertige oder gleichwirkende Elemente können in der Figur mit denselben Bezugszeichen versehen sein.

Die Figur zeigt ein Ablaufdiagramm beziehungsweise Flussdiagramm gemäß einer Ausgestaltung der vorliegenden Erfindung.

Zunächst wird in einem ersten Schritt S1 eine Messreihe für eine Schadstoffkonzentration *C*₀(*t*), eine Temperatur *T*₀(*t*), eine Windrichtung *W*₀(*t*) und/oder eine Verkehrsstärke *ρ*₀(*t*) bereitgestellt. Die Schadstoffkonzentration ist beispielsweise eine Stickoxidkonzentration. Die Schadstoffkonzentration und die Messgrößen, das heißt vorliegend die Temperatur, die Windrichtung und/oder die Verkehrsstärke wurden gemeinschaftlich erfasst. In diesem Sinne sind die Werte der Messgrößen den Werten der Schadstoffkonzentration zugeordnet. Dadurch werden für jeden Zeitpunkt, beispielsweise jede Stunde eines Tages, beispielsweise vier Werte bereitgestellt, nämlich die Schadstoffkonzentration für diesen Zeitpunkt, die Temperatur für diesen Zeitpunkt, die Windgeschwindigkeit für diesen Zeitpunkt und die Verkehrsstärke für diesen Zeitpunkt. Hierbei können durchschnittliche, gemittelte und/oder gewichtete Werte für den jeweiligen Zeitpunkt, beispielsweise über einen Zeitbereich von einer Stunde, verwendet werden.

Mit anderen Worten werden vier Zeitreihen *C*₀(*t*), *T*₀(*t*), *W*₀(*t*), *ρ*₀(*t*) bereitgestellt, wobei für jeden Zeitpunkt der Zeitreihen, ein Messwert der Schadstoffkonzentration, ein Messwert der Temperatur, ein Messwert der Windgeschwindigkeit und ein Messwert der Verkehrsstärke vorliegt. Die Messwerte müssen nicht zu diesem Zeitpunkt erfasst worden sein, sondern können repräsentativ für diesen Zeitpunkt ausgewählt oder ermittelt worden sein, beispielsweise durch eine Mittelung. Beispielsweise umfassen die Zeitreihe 24 Werte, die zu den Stunden eines Tages korrespondieren.

In einem zweiten Schritt S2 wird mittels der gemessenen Zeitreihen *T*₀(*t*), *W*₀(*t*), *ρ*₀(*t*) der Messgrößen durch ein Domain-Modell eine erste Schadstoffemission *E*₀, für wenigstens einen der Zeitpunkte *t,* bevorzugt für alle Zeitpunkte *t,* berechnet. Die erste Schadstoffemission *E*₀ basiert somit auf tatsächlichen Messwerten beziehungsweise Messdaten. Typischerweise sind hierbei Temperatur und Verkehrsstärke relevant. Die Windgeschwindigkeit ist für die Emissionen weniger relevant.

In einem dritten Schritt S3, der parallel zu S2 durchgeführt werden kann, wird wenigstens ein Wert wenigstens einer Messgröße verändert. Beispielsweise wird die zum Zeitpunkt *t* vorliegende Temperatur um 3 Prozent erhöht, und dadurch eine neue synthetische Messreihe erzeugt. Die derart neu erzeugte Zeitreihe beziehungsweise Messreihe weist wenigstens einen Wert auf, der auf dieser Änderung basiert und demnach nicht gemessen wurde. In diesem Sinne ist die durch die Veränderung erzeugte Messreihe synthetisch. Anschließend wird aus den unveränderten Zeitreihen für die Windgeschwindigkeit und die Verkehrsstärke und aus der veränderten Messreihe für die Temperatur eine zweite Schadstoffemission *E*₁, für den Zeitpunkt an welchem der Messwert der Temperatur verändert wurde, berechnet. Die zweite Schadstoffemission *E*₁ basiert somit auf tatsächlichen Messwerten beziehungsweise Messdaten und der durch die Veränderung synthetisch erzeugten Messreihe.

Nach den Schritten S2 und S3 liegen somit für wenigstens einen Zeitpunkt zwei berechnete Schadstoffemissionen *E*₀, *E*₁ vor.

In einem vierten Schritt S4 wird mittels der relativen Abweichung Δ*E*/*E*₀ = (*E*₁ - *E*₀)/*E*₀ der berechneten Schadstoffemissionen die relative Änderung der Schadstoffkonzentration mittels Δ*C*/*C*₀ = *α*Δ*E*/*E*₀ berechnet. Hierbei ist mit *α* der verkehrsbedingte Anteil an der Schadstoffkonzentration bezeichnet. Beispielsweise weist *α* den Wert 0,4 auf.

Aus der relativen Änderung der Schadstoffkonzentration (an dem betrachteten Zeitpunkt) wird mittels der Messreihe der Schadstoffkonzentration eine neue synthetische Messreihe für die Schadstoffkonzentrationen dadurch erzeugt, dass der Messwert *C*₀, der zum betrachteten Zeitpunkt vorliegt, um Δ*C* verändert wird. Dadurch wird die neue Zeitreihe (synthetische Messreihe) erzeugt, die für das Trainieren des neuronalen Netzes zusätzlich zur ursprünglich bereitgestellten gemessenen Messreihe der Schadstoffkonzentrationen verwendet werden kann. Grundsätzlich kann das obenstehend beschriebene Vorgehen für alle Zeitpunkte oder Teile der Zeitpunkte durchgeführt werden.

Im Folgenden wird ein vereinfachtes Ausführungsbeispiel erläutert.

Für einen bestimmten Zeitpunkt und einem bestimmten Ort, beispielsweise dem Ort der Messstation, liegt ein hoher Messwert der Stickoxidkonzentration, das heißt ein Messwert oberhalb des Schwellenwertes oder Grenzwertes, vor. Hierzu werden für diesen Zeitpunkt eine bestimmte Temperatur, Windrichtung und Verkehrsstärke gemessen.

Mittels des für die Schadstoffemissionen des Verkehrs spezifischen Domain-Modells wird für die gemessene Temperatur, Windrichtung und Verkehrsdichte (Eingabegrößen oder Eingabeparameter des Domain-Modells) die erste Schadstoffemission für diesen Zeitpunkt, beispielsweise 30 µg/m/s an Stickoxide, berechnet.

Anschließend wird eine weitere Berechnung mit einer leicht veränderten Temperatur, beispielsweise um 5 Prozent oder 5 Grad Celsius gegenüber der ursprünglich gemessenen Temperatur erhöht, mittels des Domain-Modells durchgeführt. Die Windgeschwindigkeit und die Verkehrsstärke bleiben hierbei unverändert. Hieraus ergibt sich die zweite Schadstoffemission, beispielsweise 33 µg/m/s an Stickoxide. Dadurch ergibt sich eine relative Änderung der Schadstoffemission um 10 Prozent. Diese relative Änderung der Schadstoffemission wird nun auf eine relative Änderung der Schadstoffkonzentration umgelegt beziehungsweise umgerechnet.

Die Schadstoffkonzentration umfasst typischerweise mehrere Anteile, beispielsweise einen Anteil des Verkehrs (verkehrsbedingter Anteil), einen Anteil der Gebäude und einen Anteil aus der Energieerzeugung. Beispielsweise ist der verkehrsbedingte Anteil *α* gleich 44 Prozent, der gebäudebedingte Anteil oder regionalbedingte Anteil 18 Prozent und der energieerzeugungsbedingte Anteil 38 Prozent. Insbesondere der verkehrsbedingte Anteil sinkt bezüglich Stickoxiden seit Jahren und wird sich voraussichtlich in den kommenden Jahren weiter reduzieren.

Aus dem verkehrsbedingten Anteil, welchen das Domain-Modell bezüglich der Schadstoffemissionen umfasst, kann dann durch Δ*C*/*C*₀ *= α*Δ*E*/*E*₀ auf die relative Änderung der Schadstoffkonzentration geschlossen werden. Bei einer relativen Änderung der Schadstoffemission um 10 Prozent ergibt sich somit eine relative Änderung der Schadstoffkonzentration um 4,4 Prozent. Das heißt, dass sich die ursprünglich gemessene Schadstoffkonzentration zum vorliegend betrachteten Zeitpunkt um 4,4 Prozent ändern würde. Mit anderen Worten übersetzt sich eine 10 prozentige Änderung der Temperatur oder eine Änderung der Temperatur um 5 Grad Celsius in eine 4,4 prozentige Änderung der Schadstoffkonzentration.

Wird das obenstehend erläuterte Verfahren für jeden Zeitpunkt oder für weitere ausgewählte Zeitpunkte der gemessenen Zeitreihe für die Schadstoffkonzentrationen durchgeführt, so kann eine neue synthetische Zeitreihe beziehungsweise Messreihe für die Schadstoffkonzentration erzeugt werden. Das neuronale Netz kann dann mit dieser neu erzeugten Zeitreihe trainiert werden.

Das beschriebene Verfahren ist computergestützt und kann mit einem Rechner, einem zentralen oder dezentralen Server, in der Cloud oder mittels eines Quantencomputers durchgeführt werden. Weiterhin basiert das computergestützte Verfahren auf Messwerten von physikalischen Messgrößen, die als Eingangsgrößen beziehungsweise Eingangsparameter einbezogen sind.

Obwohl die Erfindung im Detail durch die bevorzugten Ausführungsbeispiele näher illustriert und beschrieben wurde, so ist die Erfindung nicht durch die offenbarten Beispiele eingeschränkt oder andere Variationen können vom Fachmann hieraus abgeleitet werden, ohne den Schutzumfang der Erfindung zu verlassen.

### Bezugszeichenliste

- S1: erster Schritt
- S2: zweiter Schritt
- S3: dritter Schritt
- S4: vierter Schritt

## Patentansprüche

1. Computergestütztes Verfahren zum Erzeugen von Trainingsdaten für ein neuronales Netz, wobei das neuronale Netz zum Ermitteln einer Schadstoffkonzentration aus wenigstens einer Schadstoffemission ausgebildet ist, **gekennzeichnet durch** folgende Schritte:
- Bereitstellen wenigstens einer Messreihe der Schadstoffkonzentration mit wenigstens einem Messwert, der oberhalb eines festgelegten Schwellenwertes ist;
- Bereitstellen wenigstens einer Messreihe für eine zur gemessenen Schadstoffkonzentration zugehörigen physikalischen Messgröße, insbesondere einer Temperatur, einer Windgeschwindigkeit und/oder einer Verkehrsstärke;
- Bereitstellen eines Modells, wobei das Modell einen Zusammenhang zwischen der Messgröße und der Schadstoffemission modelliert;
- Berechnen eines ersten Wertes *E*₀ der Schadstoffemission mittels des Modells, wobei hierzu wenigstens ein zu einem Wert *C*₀ der bereitgestellten gemessenen Schadstoffkonzentration zugehöriger Messwert der Messgröße verwendet wird;
- Berechnen eines zweiten Wertes *E*₁ der Schadstoffemission mittels des Modells, wobei hierzu der für das Berechnen des ersten Wertes *E*₀ der Schadstoffemissionen verwendete Messwert der Messgröße numerisch verändert wird; und
- Erzeugen einer synthetischen Messreihe als Trainingsdaten durch eine Veränderung Δ*C* des Wertes *C*₀ der bereitgestellten gemessenen Messreihe der Schadstoffkonzentrationen, wobei die Veränderung Δ*C* mittels der relativen Änderung Δ*E*/*E*₀ der berechneten Werte der Schadstoffemissionen erfolgt.

2. Computergestütztes Verfahren gemäß Anspruch 1, **gekennzeichnet dadurch, dass** die Veränderung Δ*C* des wenigstens einen Wertes *C*₀ der bereitgestellten Messreihe der Schadstoffkonzentrationen zusätzlich mittels eines verkehrsbedingten Anteils *α* an der Schadstoffkonzentration erfolgt.

3. Computergestütztes Verfahren gemäß Anspruch 2, **gekennzeichnet dadurch, dass** die Veränderung Δ*C* des wenigstens einen Wertes *C*₀ der bereitgestellten Messreihe der Schadstoffkonzentration mittels Δ*C*/*C*₀ = *α*Δ*E*/*E*₀ erfolgt.

4. Computergestütztes Verfahren gemäß Anspruch 2 oder 3, **gekennzeichnet dadurch, dass** ein verkehrsbedingter Anteil *α* im Bereich von 0,3 bis 0,5 verwendet wird.

5. Computergestütztes Verfahren gemäß einem der vorhergehenden Ansprüche, **gekennzeichnet dadurch, dass** eine Stickoxidkonzentration als Schadstoffkonzentration und eine Stickoxidemission als Schadstoffemission verwendet wird.

6. Computergestütztes Verfahren gemäß einem der vorhergehenden Ansprüche, **gekennzeichnet dadurch, dass** als physikalische Messgröße eine Temperatur, eine Windgeschwindigkeit und/oder einer Verkehrsstärke verwendet wird.

7. Computergestütztes Verfahren gemäß einem der vorhergehenden Ansprüche, **gekennzeichnet dadurch, dass** die Messreihe der Schadstoffkonzentration und die Messreihe der Messgröße mittels einer Messstation innerhalb einer Stadt erfasst wurden.

8. Computergestütztes Verfahren gemäß einem der vorhergehenden Ansprüche, **gekennzeichnet dadurch, dass** als Modell ein domainbasiertes Modell verwendet wird.

9. Computergestütztes Verfahren zum Trainieren eines neuronalen Netzes, wobei das neuronale Netz zum Ermitteln einer Schadstoffkonzentration aus wenigstens einer Schadstoffemission ausgebildet ist, **gekennzeichnet dadurch, dass** zum Trainieren des neuronalen Netzes ein gemäß einem der vorhergehenden Ansprüche erzeugter Trainingsdatensatz verwendet wird.

10. Computergestütztes Verfahren zum Ermitteln einer Schadstoffkonzentration mittels eines neuronalen Netzes und mittels eines Modells, wobei das neuronale Netz zum Ermitteln einer Schadstoffkonzentration aus wenigstens einer Schadstoffemission ausgebildet und gemäß Anspruch 9 trainiert ist, wobei das Modell einen Zusammenhang zwischen einer physikalischen Messgröße, insbesondere einer Temperatur, einer Windgeschwindigkeit und/oder einer Verkehrsstärke, und der Schadstoffemission modelliert, **gekennzeichnet durch** folgende Schritte:
- Berechnen eines Wertes der Schadstoffemission mittels des Modells, wobei hierzu wenigstens ein Messwert der Messgröße verwendet wird; und
- Ermitteln der Schadstoffkonzentration aus dem berechneten Wert der Schadstoffemission mittels des neuronalen Netzes.

## Claims

1. Computer-aided method for generating training data for a neural network, the neural network being designed to determine a pollutant concentration from at least one pollutant emission, **characterized by** the following steps:
- providing at least one measurement series of the pollutant concentration containing at least one measured value that is above a defined threshold value;
- providing at least one measurement series for a physical measured variable associated with the measured pollutant concentration, in particular a temperature, a wind speed and/or a traffic level;
- providing a model, the model modelling a relationship between the measured variable and the pollutant emission;
- computing a first value E₀ of the pollutant emission by means of the model, this being accomplished by using at least one measured value, of the measured variable, that is associated with a value C₀ of the provided measured pollutant concentration;
- computing a second value E₁ of the pollutant emission by means of the model, this being accomplished by numerically altering the measured variable's measured value used for computing the first value E₀ of the pollutant emissions; and
- generating a synthetic measurement series as training data by way of an alteration ΔC to the value C₀ of the provided measured measurement series of the pollutant concentrations, the alteration ΔC being made by means of the relative change ΔE/E₀ in the computed values of the pollutant emissions.

2. Computer-aided method according to Claim 1, **characterized in that** the alteration ΔC to the at least one value C₀ of the provided measurement series of the pollutant concentrations is additionally made by means of a traffic-related proportion α of the pollutant concentration.

3. Computer-aided method according to Claim 2, **characterized in that** the alteration ΔC to the at least one value C₀ of the provided measurement series of the pollutant concentration is made by means of ΔC/C₀ = αΔE/E₀.

4. Computer-aided method according to Claim 2 or 3, **characterized in that** a traffic-related proportion α in the range from 0.3 to 0.5 is used.

5. Computer-aided method according to one of the preceding claims, **characterized in that** a nitrogen oxide concentration is used as the pollutant concentration and a nitrogen oxide emission is used as the pollutant emission.

6. Computer-aided method according to one of the preceding claims, **characterized in that** the physical measured variable used is a temperature, a wind speed and/or a traffic level.

7. Computer-aided method according to one of the preceding claims, **characterized in that** the measurement series of the pollutant concentration and the measurement series of the measured variable were captured by means of a measurement station within a town.

8. Computer-aided method according to one of the preceding claims, **characterized in that** the model used is a domain-based model.

9. Computer-aided method for training a neural network, the neural network being designed to determine a pollutant concentration from at least one pollutant emission, **characterized in that** a training dataset generated according to one of the preceding claims is used to train the neural network.

10. Computer-aided method for determining a pollutant concentration by means of a neural network and by means of a model, the neural network being designed to determine a pollutant concentration from at least one pollutant emission and being trained according to Claim 9, the model modelling a relationship between a physical measured variable, in particular a temperature, a wind speed and/or a traffic level, and the pollutant emission, **characterized by** the following steps:
- computing a value of the pollutant emission by means of the model, this being accomplished by using at least one measured value of the measured variable; and
- determining the pollutant concentration from the computed value of the pollutant emission by means of the neural network.

## Revendications

1. Procédé assisté par ordinateur de production de données d'apprentissage pour un réseau neuronal, dans lequel le réseau neuronal est constitué pour la détermination d'une concentration de polluant d'au moins une émission de polluant, **caractérisé par** les stades suivants :
- se procurer au moins une série de mesure de la concentration de polluant ayant au moins une valeur de mesure, qui est au-dessus d'une valeur de seuil fixée ;
- se procurer au moins une série de mesure d'une grandeur physique appartenant à la concentration de polluant mesurée, en particulier une température, une vitesse du vent et/ou une intensité du trafic ;
- se procurer un modèle, dans lequel le modèle modélise une relation entre la grandeur de mesure et l'émission de polluant ;
- calculer une première valeur ***E*₀** de l'émission de polluant au moyen du modèle, dans lequel on utilise à cet effet au moins une valeur de mesure de la grandeur de mesure appartenant à une valeur ***C*₀** de la concentration de polluant mesurée, que l'on s'est procurée ;
- calculer une deuxième valeur ***E*₁** de l'émission de polluant au moyen du modèle, dans lequel on modifie à cet effet numériquement la valeur de mesure de la grandeur de mesure utilisée pour le calcul de la première valeur ***E*₀** des émissions de polluant ; et
- produire une série de mesure synthétique sous la forme de données d'apprentissage par une modification **Δ*C*** de la valeur ***C*₀** de la série de mesure mesurée, que l'on s'est procurée, des concentrations de polluant, dans lequel la modification **Δ*C***
. s'effectue au moyen de la modification relative Δ*E*/*E*₀ des valeurs calculées des émissions de polluant.

2. Procédé assisté par ordinateur suivant la revendication 1, **caractérisé en ce que** la modification Δ*C* de la au moins une valeur *C*₀ de la série de mesure, que l'on s'est procurée, des concentrations de polluant s'effectue supplémentairement au moyen d'une proportion α, due au trafic, de la concentration de polluant.

3. Procédé assisté par ordinateur suivant la revendication 2, **caractérisé en ce que** la modification Δ*C* de la au moins une valeur *C*₀ de la série de mesure, que l'on s'est procurée, de la concentration de polluant s'effectue au moyen de Δ*C*/*C*₀ = αΔ*E*/*E*₀.

4. Procédé assisté par ordinateur suivant la revendication 2 ou 3, **caractérisé en ce que** l'on utilise une proportion α, due au trafic, dans la plage de 0,3 à 0,5.

5. Procédé assisté par ordinateur suivant l'une des revendications précédentes, **caractérisé en ce que** l'on utilise une concentration d'oxyde d'azote comme concentration de polluant et une émission d'oxyde d'azote comme émission de polluant.

6. Procédé assisté par ordinateur suivant l'une des revendications précédentes, **caractérisé en ce que** l'on utilise, comme grandeur physique, une température, une vitesse du vent et/ou une intensité du trafic.

7. Procédé assisté par ordinateur suivant l'une des revendications précédentes, **caractérisé en ce que** l'on relève la série de mesure de la concentration de polluant et la série de mesure de la grandeur de mesure au moyen d'un poste de mesure dans une ville.

8. Procédé assisté par ordinateur suivant l'une des revendications précédentes, **caractérisé en ce que** l'on utilise, comme modèle, un modèle reposant sur un domaine.

9. Procédé assisté par ordinateur d'apprentissage d'un réseau neuronal, dans lequel le réseau neuronal est constitué pour déterminer une concentration de polluant d'au moins une émission de polluant, **caractérisé en ce que**, pour faire l'apprentissage du réseau neuronal, on utilise un ensemble de données d'apprentissage produit suivant l'une des revendications précédentes.

10. Procédé assisté par ordinateur de détermination d'une concentration de polluant au moyen d'un réseau neuronal et au moyen d'un modèle, dans lequel le réseau neuronal est constitué pour la détermination d'une concentration de polluant d'au moins une émission de polluant et subit un apprentissage suivant la revendication 9, dans lequel le modèle modélise une relation entre une grandeur de mesure physique, en particulier une température, une vitesse du vent et/ou une intensité du trafic, et l'émission de polluant, **caractérisé par** les stades suivants :
- calcul d'une valeur de l'émission de polluant au moyen du modèle, dans lequel on utilise à cet effet au moins une valeur de mesure de la grandeur de mesure ; et
- détermination de la concentration de polluant, à partir de la valeur calculée de l'émission de polluant, au moyen du réseau neuronal.
.
